Europäisches Patentamt

European Patent Office

Office européen des brevets

(19)

(11) EP 1 282 061 A1

(12) EUROPEAN PATENT APPLICATION

(43) Date of publication:
05.02.2003 Bulletin 2003/06

(51) Int Cl.7: G06F 19/00, G01N 33/68

(21) Application number: 01118627.7

(22) Date of filing: 02.08.2001

(84) Designated Contracting States:
AT BE CH CY DE DK ES FI FR GB GR IE IT LI LU
MC NL PT SE TR
Designated Extension States:
AL LT LV MK RO SI

(71) Applicant: Boehringer Ingelheim International
GmbH
55216 Ingelheim am Rhein (DE)

(72) Inventors:
• Eisenhaber, Frank, Dr.
1210 Wien (AT)
• Eisenhaber, Birgit, Dr.
1210 Wien (AT)
• Maurer-Stroh, Sebastian
1030 Wien (AT)

(74) Representative: Betten & Resch
Patentanwälte,
Theatinerstrasse 8
80333 München (DE)

(54) **Method for identifying proteins with N-terminal N-myristoylation**

(57)    Two-step procedure for determining possible
N-terminal N-myristoylation of proteins from their amino
acid sequence. In the first step, a novel computerized
algorithm deriving a decision calculated from the N-ter-
minal segment of the amino acid sequence is applied to
determine probable substrate proteins. The reduced list
of targets may be subjected to experimental verification
in a second step. The score function used in the decision
does not only evaluate amino acid type preferences on
single positions, but penalizes also deviations from the
physico-chemical requirements for several positions
that either show a general trend of deviation from the
average properties of known myristoylated proteins or
have been tested for compensatory effects. For risk
evaluation, the scores are translated into probabilities
of false positive prediction.

EP 1 282 061 A1

Calculating profile sum score

$$S_{profile}$$

S1

Calculating physical properties sum score

$$S_{ppt}$$

S2

Obtaining total sum score

$$S = S_{profile} + S_{ppt}$$

S3

Converting calculated score S into probability of
false-positive prediction with generalized
extreme value distribution

S4

Fig. 1

## Description

Field of the Invention

The present invention relates to a method for identifying proteins with N-terminal N-myristoylation from the knowledge of their amino acid sequence by computational analysis.

Background of the invention

Among the many known lipid modifications N-myristoylation is one of the best investigated and has been subject to several reviews (Towler et al. 1988b; Gordon et al. 1991; Han and Martinage 1992; Johnson et al. 1994; Boutin 1997). However, after the importance for cellular regulation, signal transduction, apoptosis (Zha et al. 2000) and the potential for diverse medical treatments (Felsted, Glover, and Hartman 1995; Parang et al. 1997; Sikorski et al. 1997; Gunaratne et al. 2000) have been rediscovered and the first structures of Myristoyl-CoA:Protein N-myristoyltransferases were published (Weston et al. 1998; Bhatnagar et al. 1998), this topic has gained increasing attention.

The rare $C_{14}$ saturated fatty acid is linked most often cotranslationally (Olson and Spizz 1986; Wilcox, Hu, and Olson 1987) via an amid bond (Olson, Towler, and Glaser 1985) specific to the N-terminal glycine (Kamps, Buss, and Sefton 1985; Towler et al. 1987) of several eukaryotic and viral proteins. In general, the attachment of the lipid moiety results in an increase of hydrophobicity that plays an important role in membrane and protein association. Myristic acid represents less than 1% of all fatty acids in cells (Khandwala and Kasper 1971), but its specific length provides the possibility for reversible interactions with other proteins or membranes (Peitzsch and McLaughlin 1993) in contrast to highly stable associations facilitated by other, more hydrophobic lipid modifications.

Myristoylation can be required but need not necessarily be sufficient for membrane anchoring, as known for example for the oncoprotein p60[v-src] (Resh 1994). The list of myristoylated proteins includes various kinases, phosphatases, cytochrome $b_5$ reductase, NO synthase, the $\alpha$ subunit of many G proteins, ADP ribosylation factors, the myristoylated alanine rich C kinase substrate (MARCKS) and other membrane- or cytoskeletal-bound structural proteins, Ca2+ binding/ EF hand proteins, as well as several viral proteins.

For the viruses, the lipid modification of the proteins is often essential by directing it to the plasma membrane of the host cell or it is necessary for the assembly of the viral structure (Moscufo, Simons, and Chow 1991) and replication in general (Raulin 2000). Many viruses take part in the pathological processes directly with their myristoylated oncoproteins (Arbelaez, Bernal, and Patarca 1999).

Myristoylation is not always reduced to a simple anchoring function. The fatty acid can also fold back to domains of the acylated protein itself and extend to the outside again controlled by the binding of $Ca^{2+}$-ions acting as a switch (Ames et al. 1996). Other examples of myristoyl switches for reversible membrane association (triggered by phosphorylation or proteolytic cleavage) can be found in MARCKS (McLaughlin and Aderem 1995) and the HIV-1 Gag precursor (Zhou and Resh 1996).

The most abundant form of myristoylation is catalyzed by myristoyl-CoA:protein N-myristoyltransferase (NMT) (Raju et al. 2000) which is absolutely specific to N-terminal glycine. The proposed invention focuses solely on the recognition of protein substrates for this enzymatic activity from the substrate's amino acid sequence.

The presently ongoing massive sequencing efforts result in an enormous amount of genomic data, which requires, in the next step, the detailed characterization of the encoded proteins. The above introduction to N-myristoylation emphasizes the immense importance of this lipid modification. Therefore in the post-genomic era, knowledge of a protein being myristoylated is invaluable additional information for its functional characterization.

The experimental procedures necessary for unambiguously identifying the lipid modification, often including the incorporation of $^3$H-labeled myristic acid, are very laborious and time consuming. Additionally, the sequence similarity among related proteins is very high (often only single mutations within the region recognized by NMT) and, therefore, explicit experimental verification is often omitted.

Instead, the sequence is referred to a concordance with a consensus sequence of the myristoylation motif provided by available pattern search tools, e.g. PROSITE (Hofmann et al. 1999; Bucher and Bairoch 1994), which was last updated in April 1990. This pattern carries only a disproportionally small amount of the currently available information about the motif and produces a highly unrealistic number of positive identifications of myristoylation sites and, with its current status, even false negative predictions.

The presently available single position based algorithms (such as the search with the PROSITE pattern) cannot deal with compensatory effects. This is an important reason why predictions of the myristoylation site have been insufficiently successful to date. The sequence context is much more critical than a unique disfavored residue. For example, while a single bulky amino acid at one position might well be tolerated in the size-limited groove where substrate recognition takes place, a second one in direct neighborhood probably will not. So, the closest positions will have to compensate the extra size of the predecessor.

Furthermore, the binding of a peptide substrate to an enzyme is a highly cooperative process in the sense of an induced fit. Therefore, not only consecutive amino acid residues but also positions further apart could have an influence on the binding of each other.

Extensive kinetic measurements of substrate specificity for the yeast NMT (Towler et al. 1988b) have emphasized this importance of the sequence context which could not be reflected by a simple pattern (such as the PROSITE motif).

NMT seems to be ubiquitous among eukaryotes. However, there exists an overlapping yet distinct (Towler et al. 1988a) substrate specificity between species, which became obvious by several observations (Duronio et al. 1991). The PROSITE pattern contains no distinctive features to deal with the species-dependent substrate specificities.

Finally, using PROSITE to identify myristoylation sites is restricted to a binary decision (yes/no) without any estimation of the quality of the motif in the investigated sequence.

Due to the unsatisfactory performance of the currently available prediction tools for N-myristoylation, there is a need for an efficient and reliable tool that can significantly reduce the number of false positive predictions and that allows even large-scale database annotations.

It was an objective of the present invention to provide a reliable method (prediction tool) to identify proteins that are candidates for N-terminal myristoylation, also from large protein sequence databases.

Summary of the Invention

The solution of the problem underlying the present invention is based on the following considerations: By using approved statistical correlation methods, the characteristics for the positions in the N-terminus of all known substrates and, therefore, the obvious single position requirements for recognition by the enzyme are validated. Significance for compensatory effects are evaluated by fulfillment of the Fisher-criterion that characterizes correlation respectively independence of sequence positions.

The proposed invention allows a reasonable pre-selection of candidate proteins and a dramatic speed up in sequence database searches aimed at the identification of proteins that have not previously been known to be myristoylated.

In a first aspect, the present invention relates to a method for identifying candidate proteins with N-terminal N-myristoylation from the knowledge of their amino acid sequence, comprising the steps of

    a) computational analysis of the N-terminal segment of the primary structure of the query protein and deciding the candidate's status by applying a scoring system based on

        (i) sensitive profile extraction,
        (ii) physical property requirements,
        (iii) compensatory effects over multiple positions, and

    b) determining by experimental verification whether a protein selected in a) is a substrate for myristoyl-CoA:protein N-myristoyltransferase.

Step b) is conducted with proteins out of a reduced candidate list.

In a preferred embodiment, the scores obtained by step (a) are translated into probabilities of false-positive prediction (a1) having a generalized extreme-value distribution function. Thus, by complementing the score function with rigorous statistics in form of a generalized extreme value distribution, a quality measurement of the myristoylation signal of investigated sequences can be provided.

The method of the invention can be carried out both in single target studies (e.g. studies aiming at identifying a protein as a therapeutic target) and in large-scale biomolecular sequence database scans.

In a second aspect, the present invention relates to a composite prediction algorithm combining profile-based sum scores ($S_{profile}$; (i)) with special terms for the conserved physical properties including compensatory effects, summarized as $S_{ppt}$((ii) and (iii)).

$$S = S_{profile} + S_{ppt}$$

According to this second aspect the present invention provides a computer implemented method of identifying protein candidates for N-terminal N-myristoylation from the knowledge of the protein amino acid sequence by computational analysis of the N-terminal segment of the primary structure of a query protein, comprising the steps of determining a profile sum score $S_{profile}$ based on a profile extraction of the N-terminal segment, determining a physical

properties sum score $S_{ppt}$ based on an analysis of the physical properties of the N-terminal segment, and obtaining a myristoylation sum score $S = S_{profile} + S_{ppt}$ as a N-terminal N-myristoylation prediction function for the query protein.

Apart from a possible starting methionine, the N-terminal position of the query sequence has to be occupied with the amino acid glycine as required for myristoylation by NMT. This can also include glycines that become N-terminal after proteolytic cleavage of the query protein.

The current dataset indicates restrictions in sequence variability of substrate proteins within the N-terminal 17 residues where the major contributions come from positions 1-6 that have to fit deep in the binding pocket. Preferably, the following calculations may be executed over up to 17 residues starting from the glycine and are weighted according to their importance within the motif.

The score $S_{profile}$ is determined by comparing the amino acid sequence profile of the query protein with the sequence profile extracted from a learning set of myristoylated proteins. The profile can be calculated by any established profile technique, for instance the PSIC method. The comparison may be carried out over up to 17 positions starting from a N-terminal glycine, possibly unveiled after proteolytic cleavage, for instance of a leading methionine.

According to a particular embodiment, the physical properties sum score $S_{ppt}$ is calculated comparing the physical properties of the N-terminal segment of the query protein with those of a learning set of myristoylated proteins.

Further embodiments in the present invention are described in the dependent claims.

To take advantage of the information collected above efficiently, a scoring function, validating the quality of a sequence as motif for recognition by NMT, should not be reduced to a term evaluating amino acid type preferences on single positions independently (e.g., as in profile approaches), but also comprise physical property restrictions as well as the compensatory effects from multiple sequence positions. Hence, a composite prediction function was created that combines profile-based sum scores using the PSIC (Position Specific Independent Counts) algorithm (Sunyaev et al. 1999; $S_{profile}$) with special terms for the conserved physical properties, summarized as $S_{ppt}$.

$$S = S_{profile} + S_{ppt}$$

Implemented in a computer program, written in the C programming language, the user may choose the taxonomic parameter set and read in the sequence that should be investigated.

Then, $S_{profile}$ is calculated for the query sequence and scored according to the profile extracted from a chosen learning set of known substrates. The conserved physical properties are assumed to follow a Gauss-like distribution when retrieved from the sequences already verified to be myristoylated. Deviation from the mean for the corresponding positions in the query sequence results in a penalty for the score proportional to the extent of the deviation. Thus, if nonconformity with the physical-chemical requirements for substrate recognition is more severe, the penalty will be higher.

The overall score is the sum of $S_{profile}$ and all the physical property terms (which are negative per definition as they are penalties). To address a threshold which sequences will be processed by NMT or not, the obtained scores are compared with the scores of the learning set and the lower limit for prediction is set to the lowermost score of an experimentally verified myristoylation site. The prediction is that proteins with higher scores should also be favored substrates of NMTs. At the same time, this simple approach cannot always reflect the naturally occurring different affinities to the enzyme in the relative size of prediction function scores.

For better judgment whether further experimental verification will be feasible for a candidate sequence and estimation of the general reliability of a prediction, the scores are translated into a probability of false-positive predictions applying generalized extreme value distribution functions (Eisenhaber et al. 2001).

In sequence space, the correct motif can occur incidentally with a certain probability. The more the scores of correctly predicted sequences differ from the scores of non-myristoylated proteins, the lower will be the probability for false-positive predictions and the higher will be the credibility of the prediction. The prediction method of the invention with its current parametrization learned from the today's available sequence examples allows even large-scale database annotations with less than 5 false positive assignments among 1000 unrelated sequences with an N-terminal glycine.

Brief Description of the Drawings

The present invention and further objects, features and advantages thereof will become more readily apparent from the following description of particular embodiments of the invention with reference to the appended drawings.

Figure 1 is a flow chart showing method steps for obtaining a sum score $\underline{S}$ as prediction function for the probability of N-terminal N-myristoylation of a query protein according to an embodiment of the present invention.

Figure 2 is a flow chart showing method steps of obtaining profile and physical property information of a learning set of myristoylated proteins according to an embodiment of the present invention.

Figure 3 is a flow chart showing method steps for identifying proteins with N-terminal N-myristoylation from a large number of proteins stored in a sequence database and for consecutively refining the method by adding new verified proteins to the learning set.

Figure 4 is a hardware configuration to which the present invention may be applied.

Figure 5 is a schematic illustration of a profile matrix showing logarithmic propensity values of specific amino acids at particular sequence positions of the myristoylated protein learning set.

Figure 6 is a chart showing the correlation between the negative logarithm of experimentally derived NMT affinity constants and calculated sum scores S.

Detailed Description of Preferred Embodiments

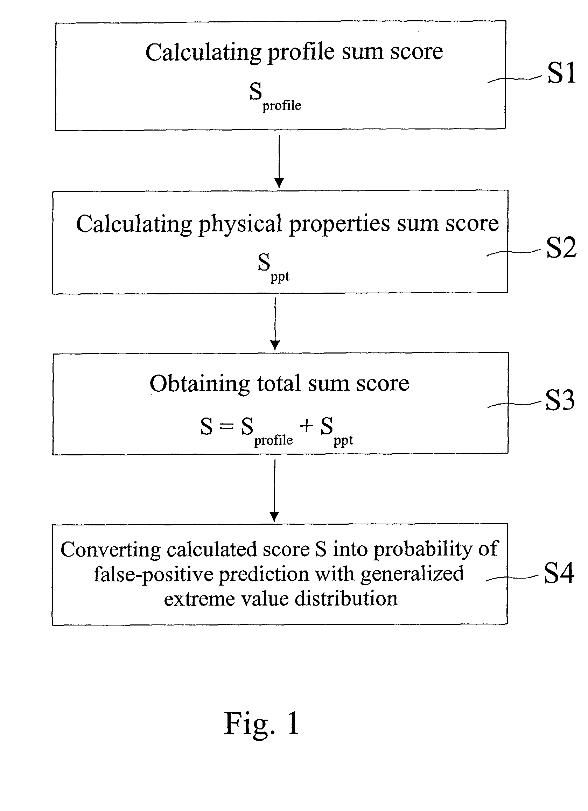

The flow chart of Figure 1 shows the method steps for obtaining the total sum score S of a query protein as a prediction function for the probability of N-terminal N-myristoylation of a query protein. In method step S1 a profile score $S_{profile}$ is calculated by sensitive profile extraction which will be explained in detail below. In the next step S2 the physical properties sum score $S_{ppt}$ is determined as will also be described in more detail below. Then the total sum score $S = S_{profile} + S_{ppt}$ is obtained in method step S3 and converted into a probability of false-positive prediction with a generalized extreme value distribution function in subsequent method step S4.

Sensitive profile extraction (i)

For a sensitive profile extraction (i), the PSIC algorithm (Sunyaev et al. 1999) may be used, which is a powerful profile extraction technique that assigns both sequence- and alignment position-specific weights (Eisenhaber, Bork, and Eisenhaber 1999; Sunyaev et al. 1999). With this technique, the corrected relative occurrences $p(a,i)$ of amino acid types $a$ at given motif positions $i$ from the gapless multiple alignment of the N-termini (except the possible starting methionines) of the complete learning set of known myristoylated proteins is determined.

It should be emphasized that this multiple alignment contains many highly similar alignments with little sequence variations often affecting only a few positions. To extract a maximum of information, an effective number $n(a,i)_{eff}$ of observations of amino acid type $a$ at alignment position $i$ is computed and finally determined $p(a,i)$ as

$$p(a,i) = \frac{n(a,i)_{eff}}{\sum_{b} n(b,i)_{eff}} \, .$$

The summation is carried out over all 20 amino acid types $b$. The value $n(a,i)_{eff}$ is thought to depend on the overall similarity of sequences having the common amino acid type $a$ in the alignment column considered. The frequency of identical alignment positions $f(a,i)$ in the subset of sequences having the same amino acid type $a$ at alignment position $i$ is used as similarity measure and is set equal to the probability of identical alignment positions for $n(a,i)_{eff}$ in random sequences. The solution of the equation

$$f(a,i) = \sum_{b} q_b^{n(a,i)_{eff}}$$

for $n(a,i)_{eff}$ estimates the number of independent observations of amino acid $a$ at position $i$ in the alignment. The value $q_b$ is the default frequency of amino acid type $b$ in a sequence database.

The final profile matrix $S_i(a)$ is calculated as (Sunyaev et al. 1999)

$$S_i(a) = \ln P \frac{P(a,i)}{q_a}.$$

These values represent the subscores of single positions *i* that sum up to regional scores within their defined regions.

$$S_{region} = \sum_{i \in region} S_i$$

The regional subscores finally enter S_profile adjusted with a weighting factor α_region, emphasizing the importance of key positions, and a normalization condition α_profile, compensating for the different lengths of the sequence regions.

$$\alpha_{profile}^{-1} \cdot profile\_length = \sum_{region} \alpha_{region} \cdot region\_length$$

The complete profile score term S_profile can therefore be circumstantiated as follows:

$$S_{profile} = \sum_{regions} \alpha_{profile} \alpha_{region} S_{region}$$

The profile is computed over about 40 amino acids following the obligatory N-terminal glycine for all entries of the learning set. The scores for query sequences are calculated only for positions 2 to 17. The latter position range corresponds to sequence segments showing detectable property deviations in N-terminally N-myristoylated proteins compared with unrelated sequences.

Physical property requirements and compensatory effects ((ii) and (iii))

The functional form of multiple residue correlation terms with respect to physical properties (P) composing S_ppt is selected in such a manner that clear deviations from value ranges in the learning set are penalized. At the same time, compliance with the consensus signal extracted from the learning set results in a zero score (but not in positive scores).

$$T_j(P) = \begin{cases} 0 & if \quad P \le P_j \\ -(P - P_j)^2 / (2\sigma_j^2) & if \quad P > P_j \end{cases}$$

Herewith, it is recognized that the form of physical terms in S_ppt may reflect the present rough understanding of requirements of the polypeptide binding site in myristoyl-CoA:protein N-myristoyltransferase. A possibly specific role of different amino acid types at certain sequence positions might be not well discerned. Definitely, it can differ among species. In its current formulation, S_ppt

$$S_{ppt} = \sum_{j=0}^{11} \alpha_j T_j$$

includes the following terms describing

- Side-chain volume limitations and mutual volume compensations for residues on positions 2 and 3, as well as compensation of bulkiness between position 7 and 9, respectively overall size limitation (positions 2 to 11) within the catalytic cleft (terms $T_0$, $T_3$ and $T_8$);

- Limited extent of hydrophobicity on positions 2 and 3, hydrophobicity compensations on positions 8, 9 and 10, as well as between positions 2 and 5 (terms $T_1$, $T_4$ and $T_7$);

- Spacer region (position 6 to 17) that should not contain too many hydrophobic residues, evaluated by the average of optimal matching hydrophobicity (term $T_2$);

- Flexibility compensations in the selective binding region of positions 3 to 5 (term $T_5$);

- Certain polarity requirements for positions 5 and 6 (term $T_6$).

Each of these nine physical conditions is weighted according to its importance and enters the sum above in form of the natural logarithm of a probability distribution function to be comparable with scores from profile computations. A Gauss-like distribution was assumed for values outside the allowed value ranges (see formula for $T_j(P)$ above!).
Furthermore, there are penalties with fixed thresholds that are also added to $S_{ppt}$:

- To exclude signalpeptide sequences, the typical structural features of their hydrophobic region are penalized when the hydrophobicity in a sequence window of 4 amino acids exceeds a certain threshold (term $T_9$ and $T_{10}$);
- Penalties are also given for extraordinary residues on special positions in concordance with the compositional analysis and the kinetic measurements (term $T_{11}$).

The purpose of introducing $S_{ppt}$ consists in excluding sequences as unlikely candidates for myristoylation due to untypical integral sequence properties compared with the learning set.

Estimation of the probability of false positive prediction (a1)

Furthermore, it was sought to facilitate the interpretation of the scores generated by the program of step a) for users that are not familiar with the prediction function and to make the obtained scores comparable with scores calculated for other features (for example prediction of GPI-anchor sites; Eisenhaber et al., 1999) This problem was solved by further applying, in a preferred embodiment of the invention, step (a1) in the framework of statistical theory by calculating the probability of false positive predictions, i.e., the probability of incidental occurrences of a motif match with the same or better score in an unrelated sequence. If a score S is normally distributed, then the probability P of a score S to be larger than a threshold $S_{th}$ is described by an extreme-value distribution (Altschul et al. 1994) with generalized analytical form (Eisenhaber et al. 2001):

$$P(S > S_{th}) = 1 - e^{-e^{-f(S_{th})}}$$

$$f(S_{th}) = \sum_{i=1}^{n} \lambda_i (S_{th} - u)^i$$

The scores for sets of unrelated sequences (non-myristoylated proteins) for the respective taxonomic groups are calculated and the best reasonable polynomial fit (*i* is between 2 and 6) is evaluated to describe the generalized extreme value distribution. So the score of the investigated sequence can be converted into a probability of false positive prediction.

The method of the invention distinguishes three groups of sequences: The first one consists of the sequences that obtain positive scores. Their probability of being false positive is maximum 0.5% for the different taxonomic parameter sets. Based on the present understanding of the requirements of the NMT binding pocket, they may be predicted as a "certain" myristoylation site. The second group can be seen as a twilight zone, because there are also verified myristoylation sites with scores between -2 and 0. They can be predicted as "probable", as the probability of having a score > -2 is accordingly higher (2,8% maximum). Sequences with scores lower than -2 represent the third group and will not be predicted by the program of the invention as protein candidates for N-myristoylation.

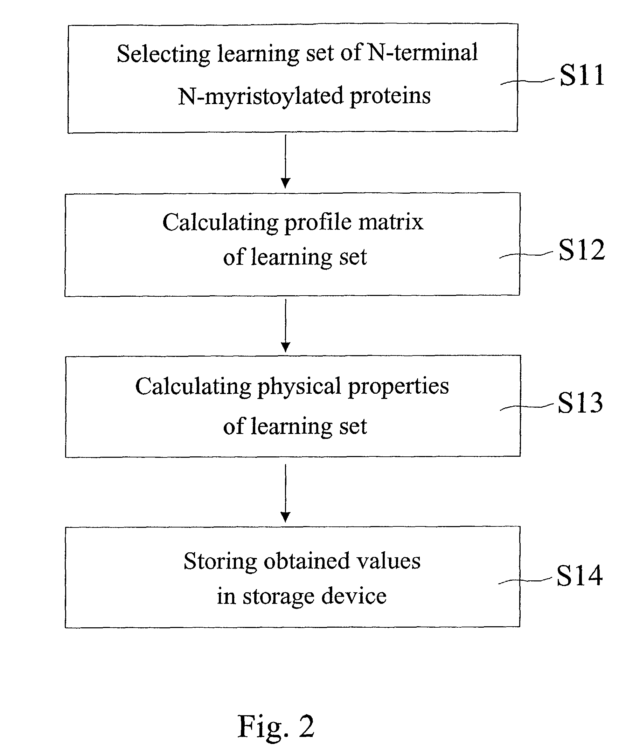

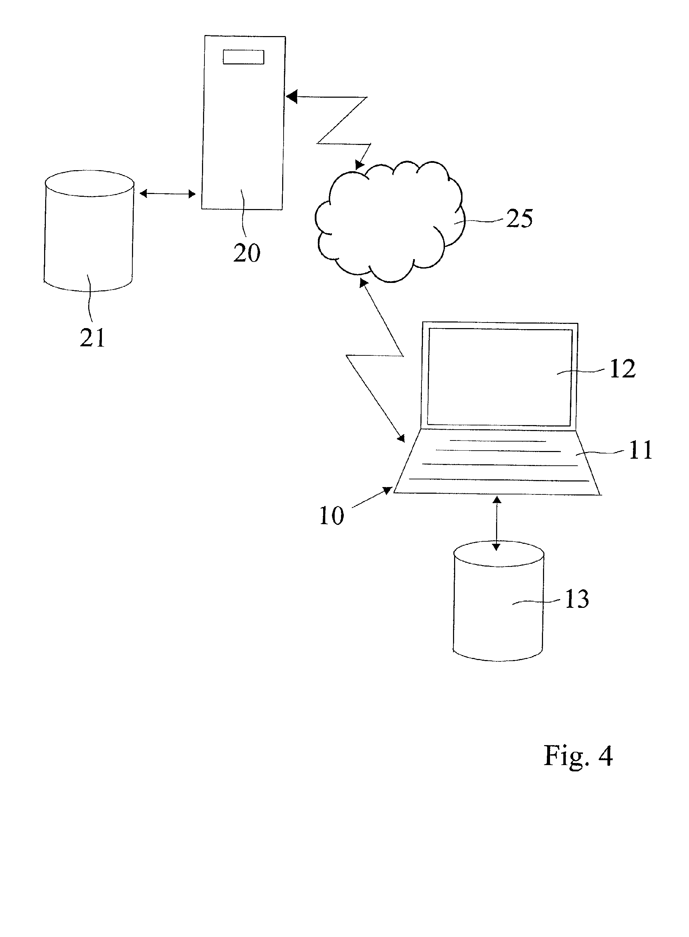

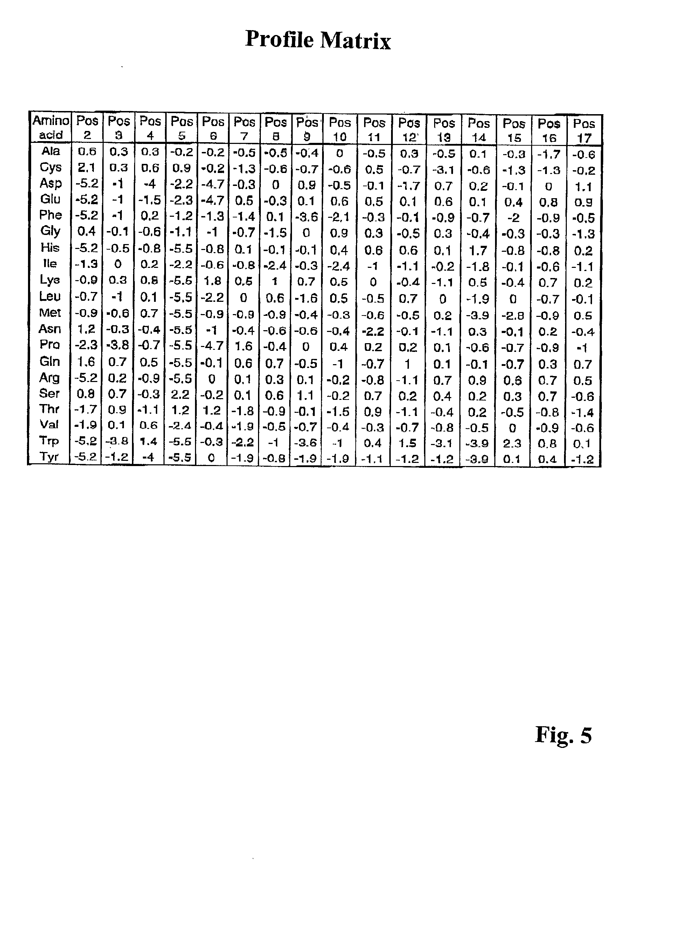

The method of obtaining the profile and physical property data of a learning set of myristoylated proteins is schemtically illustrated in the flow chart of Figure 2. In method step S11 a learning set of N-terminal N-myristoylated proteins is selected. Then, in step S12, a profile matrix (see Figure 5) is calculated for a learning set and the physical properties of the first 17 positions of the amino acid sequence of the proteins of the learning set are determined (step S13). The profile and physical property data of the myristoylated protein learning set are then stored in a storage device like storage device 13 shown in Figure 4.

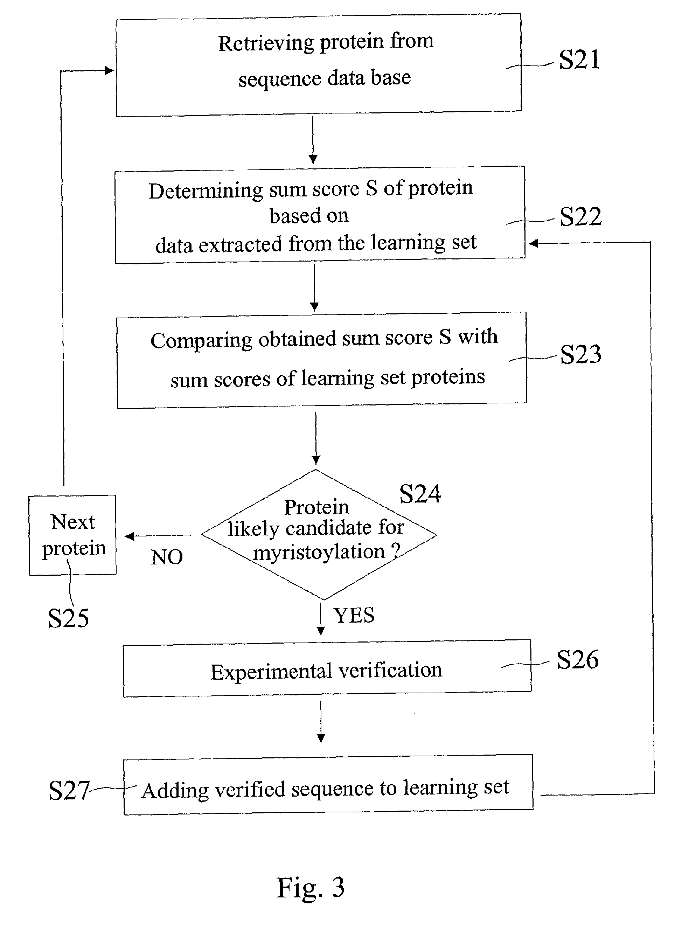

Figure 3 illustrates a method according to an embodiment of the present invention for identifying myristoylated proteins from a large number of proteins stored in a sequence database. In step S21 a first protein is retrieved from the sequence database. In subsequent method step S22 the sum score S of the protein is determined in accordance with the method illustrated in Figure 1 and based on the data extracted from a learning set (see Figure 2). The obtained sum score S is then compared with the sum scores of the learning set proteins. If the sum score S is smaller than a threshold value for which, based on the learning set data, the probability of myristoylation is minimal, it is assumed that the protein the protein retrieved from the database is not a likely candidate for myristoylation (step S24). The method then proceeds in step S25 to the next protein and method steps S21 to S24 are repeated. If the obtained sum score S is higher than the threshold value it is assumed that the protein is a likely candidate for myristoylation and an experimental verification of this finding is performed in step S26. Then, the sequence of the experimentally verified protein can be included into the learning set in step S27, thereby facilitating consequent improvement of the description of the motif and allowing self-refinement of the method.

Figure 4 is a schematic illustration of a hardware configuration for which the present invention can be applied. A calculation of the sum scores $S_{profile}$ and $S_{ppt}$ can be carried out on a suitable computing device like a desktop or laptop computer 10, workstation or a main frame computer. The computing device includes a processing unit (not shown) and an internal storage device or is connected to an external storage device 13. The computer 10 also comprises standard input/output devices like a keyboard 11, a mouse or speech processing means (both not shown) and a display screen 12. In the memory device 13 or the internal memory the obtained data from the protein learning set may be stored.

Preferably, computer device 10 is connected via a communications network 25 like an intranet or the internet with a database server 20 coupled to a database 21 like a protein sequence database. It is then possible to scan proteins from the sequence database 21 and perform the method of the present invention with the proteins retrieved from the database 21. It should be acknowledged here that the invention is not restricted to any specific type of hardware configuration. Any suitable computing, storage or data transmission devices may be employed.

Figure 5 shows a profile matrix indicating the average probability of any one of the 20 amino acids at a specific sequence position (2 to 17) of the learning set of myristoylated proteins. This profile matrix is compared with the amino acid profile of the query protein in method step S1 in Figure 1.

Experimental verification of N-myristoylation

Whether a selected protein is a substrate for myristoyl-CoA:protein N-myristoyltransferase, can be experimentally confirmed according to step b) by methods known *per se* in the art, in particular, if the number of candidates is not too large.

Several methods that are useful in the present invention to verify the attachment of myristic acid to a protein have been described in the literature. For the purpose of identifying large numbers of myristoylated proteins from different tissues and all possible organisms, an in vitro bacterial co-expression system (Duronio et al. 1990; Knoll et al. 1995) to obtain both NMT and the candidate protein in recombinant form (sequentially expressed in *Escherichia coli*) can be used (see Example 6). Although this method only indicates in vitro myristoylation of a candidate protein, it clearly states whether the protein can be a substrate for NMT.

Isolation and purification strongly depend on the characteristics of the protein (e.g. lipophilicity, subcellular compartment: membrane-bound, cytosolic...). Therefore, it is important to follow procedures that have been reported to be suitable for the properties of the protein of interest.

In order to determine the myristoylation of viral proteins, the method of (Schultz and Oroszlan 1983) (see Example 6 below) can be used.

A frequently employed protein purification method is SDS-PAGE, followed by further identification by immunoprecipitation with a specific antibody against the candidate protein. In this method, incorporation of $^3$H-labeled myristate (via addition to the medium) helps to monitor the proteins of interest during isolation and purification processes.

A more accurate method for identifying N-myristoylation is the use of FAB-MS (Fast Atom Bombardment Mass Spectrometry; (Carr et al. 1982) or combined analytical methods (Neubert and Johnson 1995), such as GC-MS (Gas Chromatography - Mass Spectrometry) or HPLC-ESI-MS (High Pressure Liquid Chromatography - Electrospray Ionization - Mass Spectrometry). In this method, N-terminal N-myristoylation is ascertained by comparing the molecular weight obtained by mass spectrometry of a protein or its fragments with calculated masses based on the amino acid sequence of the protein. The discrepancy in weights has to correspond to the mass of the myristoyl anchor.

Other appropriate examples for methods that may be employed in the present invention are the expression and characterization of calcium-myristoyl switch proteins (Zozulya, Ladant, and Stryer 1995) or ADP-ribosylation factors (Randazzo and Kahn 1995). For high-resolution structural determination of protein-linked acyl groups it is referred to (Neubert and Johnson 1995). More detail is contained in example 6.

Validation of the prediction method

The inventors executed several acknowledged tests and comparisons between predicted and experimental data to validate the methodology proposed in this invention which are listed below. Each test is explained in more detail in the following section and/or in one of the examples at the end of this document.

- Self-Consistency test (example 1)

- Jack-knife test over whole score S (example 2)

- Jack-knife test over $S_{ppt}$, while $S_{profile}$ was calculated with the whole learning set (example 2)

- Scores for proteins that are reported not to be myristoylated (example 3)

- Correlation with experimental kinetic data (example 4)

- Comparison with alternative prediction methods (example 5)

In order to clarify whether the chosen parameter sets describe the available information on N-terminal myristoylation sufficiently, it was investigated whether the chosen learning set can be predicted itself (self-consistency test).

Many prediction techniques suffer from over-parameterization; i.e., a too small learning set may be over-fitted with a too large set of parameters so that it perfectly predicts the present learning data but actually fails to describe the biological problem in its entirety. As a result, only sequences closely sequentially related to the learning set would be predicted. The so-called jack-knife or cross-validation test is designed to test for such cases. In this scheme, the predicted sequence is excluded from the learning procedure.

Two types of jack-knife tests have been applied. In the first test, the predicted sequence was excluded from the complete learning procedure (including profile derivation). In a second variant of the jack-knife test, the profile matrix calculation was executed with all sequences but the predicted sequence was left out for the calculation of $S_{ppt}$ only. Whereas the first jack-knife test checks the whole procedure for parameter over-fitting, the second test is a specific control for the parameters of the chosen physical property terms.

The scientific literature provides lists of proteins that are reported not to be myristoylated despite of their N-terminal glycine. The performance of the method of the invention was also tested for these potential candidates for false positive predictions.

Finally, the regression between affinity constants for several octapeptides towards the enzyme and the respective scores generated by the program was analyzed.

In summary, the present invention provides a novel method for the identification of N-terminal N-myristoylation that only relies on the primary structure of proteins. This technique is also useful for the selection of targets from large biomolecular sequence databases.

The central element of the invention, the novel algorithm implemented in a software tool, not only evaluates amino acid type preferences on single positions with the powerful profile extraction method PSIC, but also penalizes deviations from the physical-chemical requirements for several positions that either show a general trend of deviation from the average properties of known myristoylated proteins or have been tested for compensatory effects including multiple sequence positions. For the quantification of the risk of false positive assignment, the scores were translated into probabilities of false-positive prediction using a rigorous statistical approach. Given the high reliability of the method proposed in this invention, it becomes feasible for the first time to scan large biomolecular sequence databases and to annotate protein substrates for N-terminal myristoylation automatically. Thus, the proposed invention is a precious tool in the post-genomic era where the enormous amount of data can only be mastered with computational approaches.

Example 1

Self-consistency test

The information of the myristoylation motif of a set of test candidate proteins has been derived from a selected learning set. However, to check whether this knowledge has been converted successfully into the parameters of the prediction method of the invention, it was examined how the program would predict the learning set itself.

The program of the invention was shown to be capable to predict 359 of the 368 (97.6%) entries within the Eukaryota minus Fungi plus Viruses set. With the fungal specific parameters, which are very restrictive to maintain consistency with different substrate specificity, all 22 fungal entries (100%) were positively predicted.

The 9 entries that could not be predicted (see table 1) attract attention because they show major discrepancies with the consensus description of the motif. Several ARFs (ADP ribosylation factors) have been shown to be myristoylated (Randazzo and Kahn 1995). However, the entries listed in Table 1 do not share the motif of the experimentally verified proteins and are annotated only as potential (score lower than -2).

Penalties by the program arise because of the three consecutive large hydrophobic residues that should be hard to accommodate in the size limited pocket harboring substrate positions 2, 3 and 4. Isoleucine or valine on the critical position 5 which is situated within a narrow ring of negatively charged aspartates should also be strongly disfavored. Mutation of octapeptide GNAAAARR to GPAAAARR resulted in loss of the ability to become myristoylated (Towler et al. 1988b). Therefore, proline on 2 in STK_HYDAT (P17713) can have a similar effect by dramatically reducing the flexibility of the substrate.

The obtained results show that these 9 entries do not match the N-myristoylation consensus pattern. Therefore, it is assumed that these proteins cannot be good substrates for NMT.

Example 2

Jack-knife tests

In the first approach, the sequence that should be predicted was left out of the complete learning procedure (both profile and physical property calculations). The method used for profile extraction with sequence and position-specific weightings has the advantage that in spite of the subsets with similar sequences even small differences contribute to the extracted information. Therefore, the values calculated for the profile matrix vary even when a sequence from a subset of homologues is left out. For the large set of eukaryotes and viruses, but without fungi, 353 out of 368 (95.9%) sequences are still predicted to have a myristoylation site. From the smaller fungal set 21 of 22 (95.5%) sequences remain recognized by the program. As expected, the entries that failed to be predicted within the self-consistency test are once again out of the prediction limit. In addition, some very distinct but still trustworthy sequences produce a major shift in the profile matrix when they are left out and cannot be predicted anymore without their contribution to the profile, as expected.

The second jack-knife test shows that $S_{ppt}$ parameters are stably derived from the learning set. Therefore, the sequence that should be predicted was left out of the calculation of $S_{ppt}$ but not $S_{profile}$, resulting in a constant profile matrix throughout the whole test. The fact that for both parameter sets the prediction accuracies from the self-consistency test were reached (97.6% for EUKARYOTA-FUNGI+VIRUSES, respectively 100.0% for FUNGI) signifies that the learning set is large enough for a reliable determination of the physical property terms. At the same time, the results of the first jack-knife test show that the learning set of sequences is still too small for a stable derivation of the profile parameters. An enlarged learning set that may become available in the future will further improve the rate of correct positive prediction. It should be noted that, without making any changes to the approach outlined in this description and without modification of the associated software, an updated learning set including also protein sequences with experimentally verified N-terminal myristoylation available at a later date can be applied with the method presented.

Example 3

Scores for proteins that have been reported not to be myristoylated

One of the reviews (Boutin 1997) lists a table based on data by Tsunasawa (Tsunasawa, Stewart, and Sherman 1985) with proteins that have been investigated for myristoylation, but did not have myristic acid as attachment despite of their N-terminal glycines. Table 2 presents the scores that the method of the invention generates for these sequences. In complete concordance with the experiments, none of them would be positively predicted. (Table 2 is based on data by Tsunasawa (Tsunasawa, Stewart, and Sherman 1985) and Boutin (Boutin 1997)). It lists proteins that were reported not to be myristoylated with the scores they obtain by the algorithm of the invention. The threshold for prediction with the attribute 'probable' is a score higher than -2.0 and, thus, none of them does have a myristoylation site according to the present understanding of the motif and in agreement with the literature.

Example 4

Correlation of predicted N-terminal N-Myristoylation with experimental kinetic data

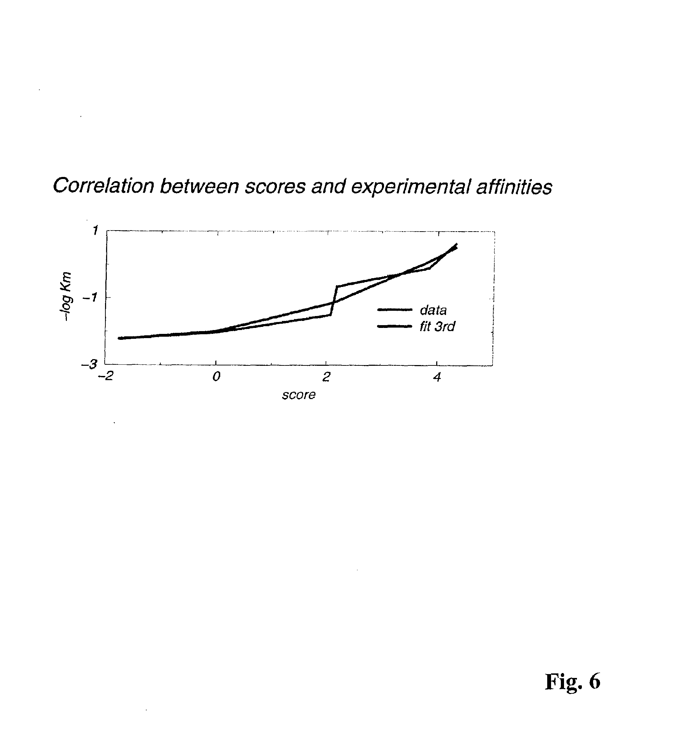

The enzyme Myristoyl-CoA:protein N-myristoyltransferase from Saccharomyces cerevisiae has been subject to extensive kinetic measurements to examine its substrate specificity (Towler et al. 1988b). Reliably quantifying the affinity constants or myristoylation velocities is a difficult task as can be seen by crude discrepancies within the provided data. For example, the $K_m$ of octapeptides GNAAAARR and GSSKSKPK were specified with 31, respectively 105 $\mu$M in one paper (Rocque et al. 1993), while an earlier publication lists other values for the same octapeptides ($K_{m\ [GNAAAARR]} = 60\ \mu$M; $K_{m\ [GSSKSKPK]} = 40\ \mu$M) (Towler et al. 1988b). It is clear that these results can only be compared within one series of experiments as they are depending on many factors (like temperature, solutions...) and experiments by different people, although from the same group, will not provide exactly the same values. However, the relation of the affinities, certifying which substrates are binding better than others, should remain constant.
In the given case, the ratio of the $K_m$ even topples, attesting GNAAAARR a 3-fold higher affinity (lower $K_m$) than GSSKSKPK, controversial to previous data where GSSKSKPK was supposed to bind better than GNAAAARR.
Nevertheless, the scores predicted by the method of the invention coincide at least qualitatively with most of the experimental data (Figure 6: Correlation between the negative logarithm of experimentally derived $K_m$ from yeast substrates and the scores calculated with the fungal parameter set). Following the more recent publication (Rocque et al. 1993) that was also used for discussion of the species dependent substrate specificities, the scores for the respective octapeptides were calculated, which all had to be extended with a common linker region. Single mutations in the derivates of cAMP-dependent protein kinase GNAAAARR cause an increase or decrease in the score proportional to the negative logarithm of the affinity. But also GARASVLS maintains the proportion, showing that not only single position deviations, but also the sequence context (after multiple mutations) is recognized at least in part by the algorithm.
Unfortunately, GLYASKLS as well as GSSKSKPK suffer from the profile parameterization because of their infrequent amino acids at position 2 to 4. Of course they are predicted positively, but their scores do not fulfill the order of affinity derived from the experiment in context of the other sequences. Finally, the score for GSSKSKPK would suit better for the values published earlier, raising the question which experimental results are more correct.
The remaining sequences however, show the expected behavior with a higher score for substrates with higher affinity to the enzyme (Figure 6).

Example 5

Comparison of the novel prediction function with commonly used prediction methods

So far, the only available tool for prediction of myristoylation sites has been the pattern search with PROSITE. Apart from its value for several other applications, it is not adequate given the complexity of this special motif. Last updated over 10 years ago, the concept of allowing only a limited subset of amino acid types on single positions will never be able to reflect the dependence on the sequence context for the recognition by NMT.
In its current status, this approach even produces false negative predictions for proteins whose myristoylation has been verified by experiment (ARF6_HUMAN [P26438], ARF6_CHICK [P26990], GCAP_BOVIN [P46065], HIA1_DICDI [P13231], HIA2_DICDI [P42526], HIPP_HUMAN [P41211], HIPP_RAT [P32076], NCAH_DROME [P42325], NECD_BOVIN [P29554], NECX_APLCA [Q16982] and many others that share broad similarity with these sequences...). Of course, updating of the PROSITE motif will correct this failure, but as a consequence also dramatically boost the number of false positives, because the allowance of further amino acids makes the weak pattern even more common.

In comparison to the predictions for proteins reported not to be myristoylated (table 2, 100% correctly not predicted), the PROSITE pattern recognition would predict myristoylation sites for 3 of these sequences (62.5% correct), respectively even 5 (only 37.5% correct) after a conscientious update.

Table 3 shows the performance of the tool of this invention compared to a PROSITE pattern search with the current motif (old, producing false negative predictions) and a corrected version (updated) over a large database. The same table lists also the number of predictions of a PROSITE pattern search over the complete SWISSALL database compared to our algorithm. For fair comparison, the PROSITE search was restricted to N-terminal glycines (possibly after a leading methionine), which is not implemented in the usual PROSITE pattern, thereby producing the highly unrealistic numbers of predictions. As PROSITE cannot distinguish between any taxon-specific substrate preferences, the prediction algorithm was reduced to the less stringent parameter set derived from a learning set containing eukaryan and viral sequences but none from fungi.

Finally, by using the information of already known myristoylated proteins restricted to certain amino acids, the PROSITE approach will never be capable of finding distinct motifs, where mutation of residues still leads to viable substrates for NMT due to compensatory effects. As can be seen from the comparison. The problem of sequence positional correlation cannot be addressed with a simple profile or pattern search alone.

Example 6

Experimental verification of the predicted myristoylation

The computational approach has the practical advantage that, among the many available proteins, examples unrelated to N-terminal myristoylation can be unselected. Thus, the resulting list of candidates becomes very small. The high-scoring hits are certain substrates for this protein modification. It is also feasible to check the remaining hits with lower scores for N-myristoylation experimentally with existing techniques.

There are several ways to verify the attachment of myristic acid to a protein. For the purpose of identifying large numbers of myristoylated proteins from different tissues and all possible organisms an in vitro bacterial coexpression system using recombinant NMT and candidate protein (sequentially expressed in *Escherichia coli*) is very efficient (Duronio et al. 1990). However, this only indicates in vitro myristoylation of a candidate protein, but nevertheless clearly states whether it can be a substrate to NMT. For details of this elaborated method see (Knoll et al. 1995).

1. Determining N-myristoylation of eukaryotic proteins by using an E. coli coexpression system (Knoll et al., 1995)

a) *E. coli* is transformed with plasmids that direct expression of NMT (Weston et al. 1998; Bhatnagar et al. 1998) and candidate protein. Next, simultaneous resistance to ampicillin and kanamycin is selected for (100 µg/ml of each in Luria broth plates).

b) Plasmid DNA from transformants is prepared and restriction endonuclease digestions used to verify that both plasmids are present.

c) A 20-ml culture of double transformant is grown at 37 °C in Luria broth plus antibiotics to $A_{600}$ of 0.5-0.7 and IPTG is added to 1 m$M$ (NMT induction).

d) The culture is incubated for 40 min, then nalidixic acid is added at 50 µg/ml (candidate protein induction).

e) 2-ml aliquots of culture are immediately added to polypropylene tubes containing [$^3$H]myristate (50-100 µCi/ml culture; label should be dried in tube under $N_2$ prior to addition of culture).

f) The cells are incubated for 30 min at 37 °C, then placed on ice for 5 min.

g) The cells are collected by centrifugation at 4000 g for 10 min and washed with 1 ml of phosphate-buffered saline (PBS). The cell suspension is transferred to a microcentrifuge tube and centrifuged once again.

h) The cells are resuspended in 50 µl lysis buffer (0.24 $M$ Tris, pH 6.8, 2% SDS/ml culture), boiled for 5 min and then centrifuged for 5 min. The supernatant is saved; total protein content is determined using BCA protein assay (Pierce, Rockford, IL). Final analysis of myristyolation is done by SDS-PAGE (load 100 µg protein/lane) and fluorography.

2. Determining N-myristoylation of viral proteins (Schultz and Oroszlan 1983):

a) Radiolabeling of cell cultures

[$^3$H]-myristic acid (0.5 mCi) is dissolved in a minimum volume of 70% ethanol and diluted to 1 ml with Eagle modified essential medium supplemented with 10% fetal calf serum. A T25 flask of each virus-producing cell line is labeled with 1 ml of this medium for 5 min. At the end of the labeling period, cells are rinsed, lysed, and immunoprecipitated with the appropriate monospecific antiserum and protein A-Sepharose (Pharmacia Fine Chemicals, Piscataway, N.J.) as described (Schultz, Rabin, and Oroszlan 1979). Sodium dodecyl sulfate-polyacrylamide gel electrophoresis (SDS-PAGE) with linear-gradient 7.5 to 17% polyacrylamide gels follows the formulation of (Laemmli 1970). Gels are impregnated with PPO (2,5-diphenyloxazole; Amersham Corp., Arlington Heights, III.) for fluorography (Laskey and Mills 1975).

b) Purification of viral proteins:

Viral proteins are labeled and purified by reverse-phase high-performance liquid chromatography as follows. Virus particles are recovered from the medium of cultures which have been labeled overnight with [$^3$H]-myristate (0.5 mCi/ml) by pelleting through a cushion of 20% sucrose in TNE buffer (0.05 M Tris-hydrochloride [pH 7.5], 150 mM NaCI, 1 mM EDTA) for 90 min at 105000$g$. The drained pellet is dissolved in 6 M guanidine hydrochloride, adjusted to pH 2 with trifluoroacetic acid, and applied to a μ Bondapak $C_{18}$ column (Waters Associates, Milford, Mass.). Gradient elution chromatography is accomplished with a Waters Associates model 660 solvent programmer, two model 6000M solvent delivery pumps, and a model 450 variable wavelength detector set at 206 nm. Solvents for reverse-phase high-performance liquid chromatography are 0.1% trifluoroacetic acid or propanol with 0.1% trifluoroacetic acid as the organic phase. Proteins are eluted in a 1-h linear gradient from 0 to 60% acetonitrile at ambient temperature followed by a 15-min linear gradient from 20 to 60 % propanol at 50°C (Henderson, Sowder, and Oroszlan 1981).

**Claims**

1. A method for identifying proteins with N-terminal N-myristoylation from the knowledge of their amino acid sequence, comprising the steps of

    (a) computational analysis of the N-terminal segment of the primary structure of the query protein and deciding the candidate's status by applying a scoring system based on

        (i) sensitive profile extraction,

        (ii) physical property requirements,

        (iii) compensatory effects over multiple positions, and

    (b) determining by experimental verification whether a protein selected in a) is a substrate for myristoyl-CoA: protein N-myristoyltransferase.

2. The method of claim 1, wherein the scores obtained in step a) are translated into probabilities of false-positive prediction with a generalized extreme value distribution.

3. A computer implemented method of identifying protein candidates for N-terminal N-myristoylation from the knowledge of the protein amino acid sequence by computational analysis of the N-terminal segment of the primary structure of a query protein, comprising the steps of:

    - determining a profile sum score $S_{profile}$ based on a profile extraction of the N-terminal segment,
    - determining a physical properties sum score $S_{ppt}$ based on an analysis of the physical properties of the N-terminal segment, and
    - obtaining a myristoylation sum score $S = S_{profile} + S_{ppt}$ as a N-terminal N-myristoylation prediction function for the query protein.

4. The method of claim 3, wherein the profile sum score $S_{profile}$ is determined by comparing the amino acid sequence profile of the query protein with the mean amino acid sequence profile calculated from a learning set of myristoylated proteins.

**5.** The method of claim 4, wherein the comparison is carried out over up to 17 positions starting from a N-terminal glycine.

**6.** The method of claim 4 or 5, including using a PSIC (position specific independent counts) algorithm for determining the profile sum score $S_{profile}$.

**7.** The method of one of claims 3 to 6, wherein the step of determining the physical properties sum score $S_{ppt}$ includes taking into account compensatory effects over multiple sequence positions.

**8.** The method of one of claims 3 to 7, wherein the step of determining the physical properties sum score $S_{ppt}$ is performed by comparing the physical properties of the amino acid sequence of the query protein with physical properties of the amino acid sequences of the learning set of myristoylated proteins.

**9.** The method of claim 8, wherein the physical properties sum score $S_{ppt}$ reflects the physical requirements of the polypeptide binding site in myristoyl-CoA:protein N-myristoyltransferase.

**10.** The method of claim 9, wherein the physical conditions for determining the physical properties sum score $S_{ppt}$ are weighted according to their respective importance.

**11.** The method of one of claims 8 to 10, wherein the step of determining the physical properties sum score $S_{ppt}$ includes a assigning a plurality of physical properties P of the query protein sequence a value $T_j (P)$ assuming that $T_j (P)$ is a logarithm of a statistical distribution density function with parameters calculated from the learning set of myristoylated proteins.

**12.** The method of one of claims 7 to 11, wherein the determination of the physical properties sum score $S_{ppt}$ is based on a calculation of the physical properties of sequence positions 2 to 17 of the query protein.

**13.** The method of one of claims 3 to 12, wherein the single or combined physical properties include side-chain volume limitations, hydrophobicity/poiarity requirements, flexibility requirements and/or mutual compensations thereof.

**14.** The method of one of claims 3 to 13, comprising the step of comparing the obtained myristoylation sum score S = $S_{profile}$ + $S_{ppt}$ of the query protein with the respective sum scores of the proteins of a myristoylated protein learning set.

**15.** The method of one of claims 3 to 14, comprising the step of converting the obtained myristoylation sum score S into probabilities of a false-positive prediction of protein myristoylation with a generalized extreme value distribution function.

**16.** The method of one of claims 3 to 15, comprising scanning a large number of query proteins from a sequence database.

**17.** The method of one of claims 3 to 15, comprising obtaining a N-terminal N-myristoylation prediction function of a single target protein sequence.

**18.** The method of claim 4, 8 or 14 or claims dependent on one of these claims, comprising updating the myristoylated protein learning set and the profile and physical property data obtained therefrom.

**19.** A computer program comprising executable program code for carrying out a method according to one of claims 1 to 18.

**20.** A data carrier having stored thereon a computer program comprising executable code for identifying protein candidates for N-terminal N-myristoylation from the knowledge of the protein amino acid sequence by computational analysis of the N-terminal segment of the primary structure of a query protein, comprising the steps of:

- determining a profile sum score $S_{profile}$ based on a profile extraction of the N-terminal segment,
- determining a physical properties sum score $S_{ppt}$ based on an analysis of the physical properties of the N-terminal segment, and
- obtaining a myristoylation sum score S = $S_{profile}$ + $S_{ppt}$ as a N-terminal N-myristoylation prediction function for

the query protein.

Calculating profile sum score

$$S_{profile}$$

S1

Calculating physical properties sum score

$$S_{ppt}$$

S2

Obtaining total sum score

$$S = S_{profile} + S_{ppt}$$

S3

Converting calculated score S into probability of false-positive prediction with generalized extreme value distribution

S4

Fig. 1

Selecting learning set of N-terminal
N-myristoylated proteins — S11

Calculating profile matrix
of learning set — S12

Calculating physical properties
of learning set — S13

Storing obtained values
in storage device — S14

Fig. 2

```
┌─────────────────────────────────────────┐
│         Retrieving protein from          │──── S21
│          sequence data base              │
└─────────────────────────────────────────┘
                    │
                    ▼
┌─────────────────────────────────────────┐
│   Determining sum score S of protein     │
│               based on                   │──── S22
│   data extracted from the learning set   │
└─────────────────────────────────────────┘
                    │
                    ▼
┌─────────────────────────────────────────┐
│   Comparing obtained sum score S with    │──── S23
│   sum scores of learning set proteins    │
└─────────────────────────────────────────┘
                    │
                    ▼
                          S24
           ◇ Protein likely candidate for
   ┌──────┐  myristoylation ? ◇
   │ Next │◄── NO
   │protein│
   └──────┘
     S25              │ YES
                      ▼
┌─────────────────────────────────────────┐
│        Experimental verification         │──── S26
└─────────────────────────────────────────┘
                    │
                    ▼
┌─────────────────────────────────────────┐
│ S27 ─ Adding verified sequence to learning set │
└─────────────────────────────────────────┘
```

Fig. 3

Fig. 4

# Profile Matrix

| Amino acid | Pos 2 | Pos 3 | Pos 4 | Pos 5 | Pos 6 | Pos 7 | Pos 8 | Pos 9 | Pos 10 | Pos 11 | Pos 12 | Pos 13 | Pos 14 | Pos 15 | Pos 16 | Pos 17 |
|---|---|---|---|---|---|---|---|---|---|---|---|---|---|---|---|---|
| Ala | 0.6 | 0.3 | 0.3 | -0.2 | -0.2 | -0.5 | -0.5 | -0.4 | 0 | -0.5 | 0.3 | -0.5 | 0.1 | -0.3 | -1.7 | -0.6 |
| Cys | 2.1 | 0.3 | 0.6 | 0.9 | -0.2 | -1.3 | -0.6 | -0.7 | -0.6 | 0.5 | -0.7 | -3.1 | -0.6 | -1.3 | -1.3 | -0.2 |
| Asp | -5.2 | -1 | -4 | -2.2 | -4.7 | -0.3 | 0 | 0.9 | -0.5 | -0.1 | -1.7 | 0.7 | 0.2 | -0.1 | 0 | 1.1 |
| Glu | -5.2 | -1 | -1.5 | -2.3 | -4.7 | 0.5 | -0.3 | 0.1 | 0.6 | 0.5 | 0.1 | 0.6 | 0.1 | 0.4 | 0.8 | 0.9 |
| Phe | -5.2 | -1 | 0.2 | -1.2 | -1.3 | -1.4 | 0.1 | -3.6 | -2.1 | -0.3 | -0.1 | -0.9 | -0.7 | -2 | -0.9 | -0.5 |
| Gly | 0.4 | -0.1 | -0.6 | -1.1 | -1 | -0.7 | -1.5 | 0 | 0.9 | 0.3 | -0.5 | 0.3 | -0.4 | -0.3 | -0.3 | -1.3 |
| His | -5.2 | -0.5 | -0.8 | -5.5 | -0.8 | 0.1 | -0.1 | -0.1 | 0.4 | 0.6 | 0.6 | 0.1 | 1.7 | -0.8 | -0.8 | 0.2 |
| Ile | -1.3 | 0 | 0.2 | -2.2 | -0.6 | -0.8 | -2.4 | -0.3 | -2.4 | -1 | -1.1 | -0.2 | -1.8 | -0.1 | -0.6 | -1.1 |
| Lys | -0.9 | 0.3 | 0.8 | -5.5 | 1.8 | 0.5 | 1 | 0.7 | 0.5 | 0 | -0.4 | -1.1 | 0.5 | -0.4 | 0.7 | 0.2 |
| Leu | -0.7 | -1 | 0.1 | -5.5 | -2.2 | 0 | 0.6 | -1.6 | 0.5 | -0.5 | 0.7 | 0 | -1.9 | 0 | -0.7 | -0.1 |
| Met | -0.9 | -0.6 | 0.7 | -5.5 | -0.9 | -0.9 | -0.9 | -0.4 | -0.3 | -0.6 | -0.5 | 0.2 | -3.9 | -2.8 | -0.9 | 0.5 |
| Asn | 1.2 | -0.3 | -0.4 | -5.5 | -1 | -0.4 | -0.6 | -0.6 | -0.4 | -2.2 | -0.1 | -1.1 | 0.3 | -0.1 | 0.2 | -0.4 |
| Pro | -2.3 | -3.8 | -0.7 | -5.5 | -4.7 | 1.6 | -0.4 | 0 | 0.4 | 0.2 | 0.2 | 0.1 | -0.6 | -0.7 | -0.9 | -1 |
| Gln | 1.6 | 0.7 | 0.5 | -5.5 | -0.1 | 0.6 | 0.7 | -0.5 | -1 | -0.7 | 1 | 0.1 | -0.1 | -0.7 | 0.3 | 0.7 |
| Arg | -5.2 | 0.2 | -0.9 | -5.5 | 0 | 0.1 | 0.3 | 0.1 | -0.2 | -0.8 | -1.1 | 0.7 | 0.9 | 0.6 | 0.7 | 0.5 |
| Ser | 0.8 | 0.7 | -0.3 | 2.2 | -0.2 | 0.1 | 0.6 | 1.1 | -0.2 | 0.7 | 0.2 | 0.4 | 0.2 | 0.3 | 0.7 | -0.6 |
| Thr | -1.7 | 0.9 | -1.1 | 1.2 | 1.2 | -1.8 | -0.9 | -0.1 | -1.5 | 0.9 | -1.1 | -0.4 | 0.2 | -0.5 | -0.8 | -1.4 |
| Val | -1.9 | 0.1 | 0.6 | -2.4 | -0.4 | -1.9 | -0.5 | -0.7 | -0.4 | -0.3 | -0.7 | -0.8 | -0.5 | 0 | -0.9 | -0.6 |
| Trp | -5.2 | -3.8 | 1.4 | -5.5 | -0.3 | -2.2 | -1 | -3.6 | -1 | 0.4 | 1.5 | -3.1 | -3.9 | 2.3 | 0.8 | 0.1 |
| Tyr | -5.2 | -1.2 | -4 | -5.5 | 0 | -1.9 | -0.8 | -1.9 | -1.9 | -1.1 | -1.2 | -1.2 | -3.9 | 0.1 | 0.4 | -1.2 |

**Fig. 5**

Correlation between scores and experimental affinities

Fig. 6

**European Patent Office**

## EUROPEAN SEARCH REPORT

Application Number

EP 01 11 8627

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | HAN K-K A ET AL: "POSSIBLE RELATIONSHIP BETWEEN CODING RECOGNITION AMINO ACID SEQUENCE MOTIF OR RESIDUES AND POST-TRANSLATIONAL CHEMICAL MODIFICATION OF PROTEINS" INTERNATIONAL JOURNAL OF BIOCHEMISTRY, vol. 24, no. 9, 1992, pages 1349-1363, XP000645190 ISSN: 0020-711X * abstract * * page 1354, right-hand column, line 31 - page 1355, right-hand column, line 3 * * page 1355; table 8 * | 1-20 | G06F19/00 G01N33/68 |
| Y  A | DATABASE PROSITE 'Online! EXPASY; October 1989 (1989-10) "N-myristoylation site" retrieved from WWW.EXPASY.ORG Database accession no. PS00008 XP002204493 * the whole document * | 1-20 | |
| Y | QI QUNGANG ET AL: "Molecular cloning, genomic organization, and biochemical characterization of myristoyl-CoA:protein N-myristoyltransferase from Arabidopsis thaliana." JOURNAL OF BIOLOGICAL CHEMISTRY, vol. 275, no. 13, 31 March 2000 (2000-03-31), pages 9673-9683, XP002204491 ISSN: 0021-9258 * abstract * * page 9673, right-hand column, line 27 - line 44 * * page 9675, left-hand column, line 66 - right-hand column, line 20 * | 1,2,19 | TECHNICAL FIELDS SEARCHED (Int.Cl.7)  G06F G01N |

-/--

The present search report has been drawn up for all claims

| Place of search | Date of completion of the search | Examiner |
|---|---|---|
| THE HAGUE | 3 July 2002 | Tuynman, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | European Patent Office | EUROPEAN SEARCH REPORT | Application Number<br>EP 01 11 8627 |

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (Int.Cl.7) |
|---|---|---|---|
| Y | EISENHABER BIRGIT ET AL: "Prediction of potential GPI-modification sites in proprotein sequences."<br>JOURNAL OF MOLECULAR BIOLOGY,<br>vol. 292, no. 3, pages 741-758,<br>XP002204269<br>ISSN: 0022-2836<br>* the whole document * | 3-20 | |
| A | TOWLER D A ET AL: "THE BIOLOGY AND ENZYMOLOGY OF EUKARYOTIC PROTEIN ACYLATION"<br>ANNUAL REVIEW OF BIOCHEMISTRY,<br>1988, pages 69-100, XP008005416<br>ISSN: 0066-4154<br>* page 78, line 33 - page 88, line 37 * | 1-20 | |
| D,A | SUNYAEV SHAMIL R ET AL: "PSIC: Profile extraction from sequence alignments with position-specific counts of independent observations."<br>PROTEIN ENGINEERING,<br>vol. 12, no. 5, May 1999 (1999-05), pages 387-394, XP002204492<br>ISSN: 0269-2139<br>* the whole document * | 1-20 | TECHNICAL FIELDS<br>SEARCHED (Int.Cl.7) |
| T | MAURER-STROH SEBASTIAN ET AL: "N-terminal N-myristoylation of proteins: Prediction of substrate proteins from amino acid sequence."<br>JOURNAL OF MOLECULAR BIOLOGY,<br>vol. 317, no. 4, 2002, pages 541-557,<br>XP002204270<br>5 April, 2002<br>ISSN: 0022-2836<br>* the whole document * | 1-20 | |

The present search report has been drawn up for all claims

| Place of search | Date of completion of the search | Examiner |
|---|---|---|
| THE HAGUE | 3 July 2002 | Tuynman, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document